# EUROPEAN PATENT SPECIFICATION

(11) **EP 1 191 926 B1**
(45) Date of publication and mention of the grant of the patent: **30.03.2005**
(21) Application number: 00946614.5
(22) Date of filing: 20.06.2000
(51) Int. Cl.: A61K 9/36, A61P 1/04, A61K 31/4439

(54) **NEW OMEPRAZOLE AND (S)-OMEPRAZOLE FORMULATION**
NEUE OMEPRAZOLE UND (S)-OMEPRAZOLE ZUSAMMENSETZUNG
NOUVELLE FORMULATION DE OMEPRAZOLE ET (S)-OMEPRAZOLE

(30) Priority: 22.06.1999 SE 9902386
(43) Date of publication of application: 03.04.2002
(73) Proprietor: AstraZeneca AB, 151 85 Södertälje (SE)
(72) Inventor: LUNDBERG, Per, Johan, S-431 83 Mölndal (SE); SJÖBLOM, Brita, S-431 83 Mölndal (SE)
(86) International application number: PCT/SE2000/001310
(87) International publication number: WO 2000/078293

(56) References cited:
- EP-A2- 0 237 200
- WO-A1-00/09092
- WO-A1-95/01783
- WO-A1-97/25979
- WO-A1-98/19668

## Description

### Field of the invention

The present invention relates to new oral pharmaceutical dosage forms comprising as active ingredient omeprazole, an alkaline salt of omeprazole, *S*-omeprazole or an alkaline salt of *S*-omeprazole. The dosage form comprises a core material of the active ingredient, one or more alkaline additives, and one or more swelling agents, wherein the core material is covered with a semipermeable membrane and without an enteric coating. Furthermore. the invention refers to the manufacture of such dosage forms and their use in medicine.

### Background of the invention and prior art.

The acid labile H⁺, K⁺-ATPase inhibitor known under the generic name omeprazole is disclosed in EP-0005129. Certain salts of omeprazole are described in EP-124495, a magnesium salt of omeprazole is described in WO 95/01977, and the single enantiomers of omeprazole and certain salts thereof are described in WO 94/27988.

Omeprazole is useful for inhibiting gastric acid secretion in mammals including man by controlling gastric acid secretion at the final step of the acid secretory pathway and thus reduce basal and stimulated gastric acid secretion irrespective of stimulus. In a more general sense, omeprazole may be used for prevention and treatment of gastric-acid related diseases in mammals and man, including e.g. reflux oesophagitis, gastritis, duodenitis, gastric ulcer, duodenal ulcer and Zollinger-Ellison syndrome. Furthermore, it may be used for treatment of other gastrointestinal disorders where gastric acid inhibitory effect is desirable e.g. in patients on NSAID therapy, in patients with Non Ulcer Dyspepsia, and in patients with symptomatic gastro-oesophageal reflux disease (GORD). Omeprazole may also be used in patients in intensive care situations, in patients with acute upper gastrointestinal bleeding, pre-and post-operatively to prevent aspiration of gastric acid and to prevent and treat stress ulceration. Further, it may be useful in the treatment of psoriasis as well as in the treatment of *Helicobacter* infections and diseases related to these where therapeutic control of gastric acid secretion is fundamental in the treatment.

Omeprazole is, however, susceptible to degradation or transformation in acidic and neutral media. The stability of omeprazole is also affected by moisture, heat, organic solvents and to some degree by light. With respect to the stability properties of omeprazole, it is established that an oral solid dosage form must be protected from contact with the acidic gastric juice and that omeprazole must be transferred in intact form to that part of the gastrointestinal tract where pH is near neutral and where rapid absorption can occur.

A pharmaceutical dosage form of omeprazole is best protected from contact with acidic gastric juice by an enteric coating layer. For instance, US 4,786,505 describes such enteric coated formulations. These formulations have a core comprising an alkaline salt of the drug or a core comprising the drug together with an alkaline reacting compound, the core is coated with a water soluble or in water rapidly disintegrating separating layer and further with an enteric coating layer. There are numerous published patent applications from different companies describing enteric coated formulations comprising omeprazole or other proton pump inhibitor compounds.

WO 96/01623 describes tableted dosage forms of omeprazole, wherein enteric coating layered pellets are compressed into a multiple unit tableted dosage form. It is essential in these tableted formulations that the enteric coating layer can withstand the compression forces.

WO97/25979 A1 is directed to a delivery system for targeted delivery to specific locations in the alimentary canal and comprises a core and a coating. According to WO97/25979 A1, the core is comprised of a drug in combination with a carrier material. One possible embodiment of WO97/25979 A1 contemplates that the carrier material swells upon contact with an aqueous medium such as that found in the alimentary canal. However, WO97/25979 A1 does not have any disrupting semipermeable membrane of a water-insoluble polymer and talc or fumed silica. Furthermore, WO97/25979 A1 does not teach or suggest the use of an alkaline additive in the core.

There are different technologies and pharmaceutical formulations described in the prior art which provide a delayed release of an administered drug. Such formulations are for instance based on osmotic differences, slow-eroding/dissolving layers, diffusion through a membrane, time controlled explosion systems or any combinations thereof. In the following some of these principles are exemplified. For instance, US 4 871 549 describes a time controlled explosion system. Conte et al (Drug Development and Industrial Pharmacy, 1989, vol. 15, pp. 2583 -96) describes a three-layer tablet giving a double pulsed system suitable for ibuprofen. US 5 567 441 describes a dosage form for diltiazem comprising a mixture of one fraction of slow release pellets and another fraction of delayed pulse release membrane coated pellets. WO97/02020 describes a dosage form of pantoprazole in combination with antibacterial substances wherein one part of the pantoprazole dose is in slow release form with a continuously release during time. US 5 178 867 describes a dosage form with an exit port or hole that connects the interior of the dosage form with the exterior.

### Summary of the invention

The present invention provides - in contrast to earlier presented oral dosage forms for proton pump inhibitor compounds - a dosage form without an enteric coating layer.

The dosage form according to the present invention comprises a core material coated with a semipermeable membrane. The core material contains an active ingredient selected from omeprazole, an alkaline salt thereof, *S*-omeprazole or an alkaline salt thereof, one or more alkaline additives, and one or more swelling agents. The semipermeable membrane, comprising a water-insoluble polymer and talc or fumed silica, wherein the talc or fumed silica and water-insoluble polymer are in a weight ratio of between 90:10 and 50:50, is able to disrupt or may change its permeability after a pre-determined time. One or more swelling agents are placed in the core material to effectuate a disruption or an increased permeability of the semipermeable membrane after such a suitable time. Optionally pharmaceutically acceptable excipients such as an osmotic agent may also be included in the core material.

Surprisingly, the formulation according to the present invention is prepared without an enteric coating, which previously have been almost an axiom for dosage forms containing omeprazole or any other proton pump inhibitor compounds. The present invention also provides the possibility to avoid the separating layer needed under an enteric coating layer to separate omeprazole from the enteric coating polymer. Omeprazole should preferably not be in contact with the esztetic coating due to discoloration and degradation of omeprazole. Thus, the present invention provides a simplified process than previous manufacture processes requesting double coating layers on the core material. See for instance, EP 247 983.

According to a further aspect of the present invention, the dosage form may preferably be in the form of a multiple unit pellet system. The prepared core material, in the form of small pellets coated with a semipermeable membrane and without an enteric coating may be filled into a capsule or compressed into a multiple unit tablet.

The core material comprises an alkalizing agent, that is sufficiently alkaline and is present in a sufficiently high amount. The core material also comprises a swelling agent that upon contact with moisture starts to swell. When the coated pellets pass the stomach small amounts of gastric fluid will be absorbed through the semipermeable membrane. The alkalizing agent in the core material will neutralize the absorbed acidic fluid and protect the active ingredient against degradation. At the same time the swelling agent, will be exposed to the penetrating fluid or moisture, and it will start to expand. After a pre-determined time interval this expansion leads to disruption of the superimposed semipermeable membrane by the built-up pressure or to a swelling that will increase the permeability of the membrane. The time interval is to be determined so that the pellets have had time to pass the stomach at that very moment, and have reached the small intestines. The entire dose of the active ingredient will then start to be released into the small intestine where absorption can occur.

### Detailed description of the drawings

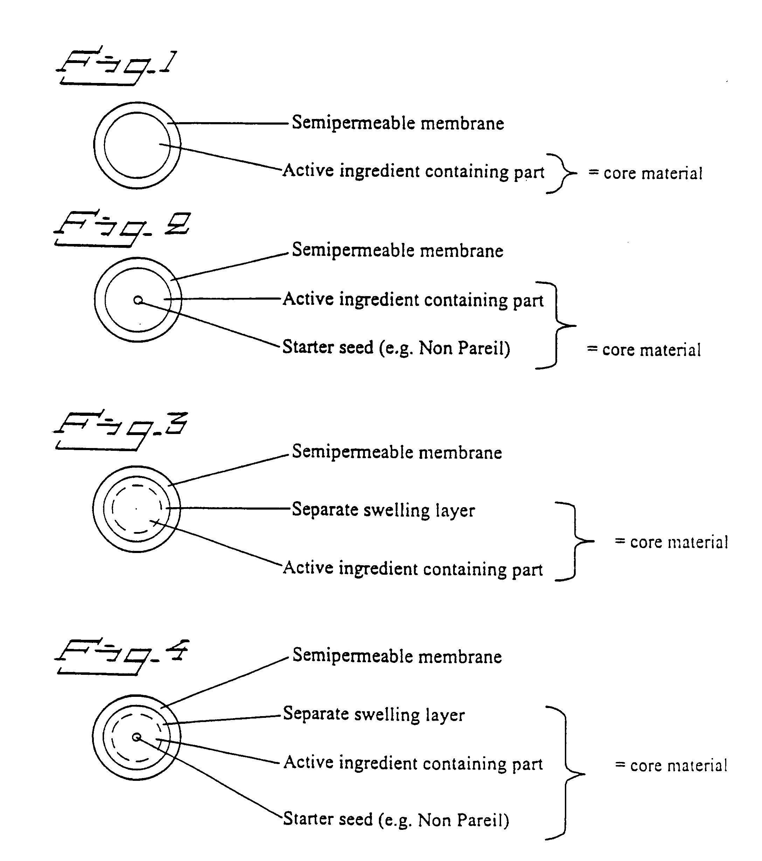

Figures 1 - 4 illustrate principles for construction of dosage forms according to the present invention. The invention comprises a core material layered with a semipermeable membrane. The core material can be prepared according to at least four different principles as shown in the Figures. The drawings are not intended to illustrate the size or relative sizes of the dosage form or its different parts.

### Detailed description of the invention

The present invention provides a core material in the form of pellets or small tablets coated with a semipermeable membrane. The composition of the core material protects the active ingredient against the gastric fluid, which permeates through the semipermeable coating during the pellet's passage through the stomach. Such pellet formulations are generally emptied from the stomach within 2-4 hours. When the pellets have left the stomach, the semipermeable membrane covering the individual pellets disrupts and/or starts to release the active ingredient in the small intestine.

The pellets coated with the semipermeable membrane may be filled into capsules prepared from gelatine or hydroxypropyl methylcellulose (HPMC), be filled into sachets or be mixed with tablet excipients and compressed to a fast disintegrating tablet or to an effervescent tablet.

### Core material

The core material may be produced with starter seeds, for instance sugar spheres like Non-pareils™, by layering the active ingredient on the seeds by conventional technique or by the use of a centrifugal granulator/ roto granulator. Alternatively, the core material has a homogenous distribution of the active agent and excipients, and is prepared e.g. by extrusion and spheronization, or by compression. Other conventional techniques known in the art are also suitable in preparing the core material.

The core material is in the form of pellets, spheroids or small tablets. The size of the formulated core materials is approximately between 0.1 and 4 mm, and preferably the core material has a diameter of 0.2 to 2.5 mm.

The core material comprises the active ingredient, an alkalizing agent, a swelling agent and optionally binders, osmotic agents and other pharmaceutically acceptable excipients.

The active ingredient is selected from the group consisting of omeprazole, an alkaline salt thereof, *S*-omeprazole or an alkaline salt thereof. Suitable alkaline salts are for instance the Mg²⁺, Ca²⁺, Na⁺, K⁺ salts, preferably the Mg²⁺ salts in a highly crystalline form. A preferred magnesium salt of omeprazole having a crystallinity of more than 70% determined by X-ray powder diffraction is described in WO95/01977, hereby incorporated by references.

Before the seeds are layered, the active ingredient may be mixed with further components to obtain preferred handling and processing properties and a suitable concentration of the active ingredient in the final mixture.

Such further components can be binders, surfactants, fillers or other pharmaceutically acceptable ingredients, alone or in mixtures. The binders are for example cellulose derivatives such as hydroxypropyl methylcellulose, methylcellulose, hydroxypropyl cellulose and carboxymethyl-cellulose sodium, and others such as polyvinyl pyrrolidone, gelatine, sugars, starches or other pharmaceutically acceptable substances with cohesive properties. Suitable surfactants are found in the groups of pharmaceutically acceptable non-ionic surfactants, such as polysorbate 80, or ionic surfactants such as for instance sodium lauryl sulphate.

An alkalizing agent is incorporated in the core material together with the active ingredient and/or the swelling agent, preferably together with the active ingredient. The alkalizing agent is present in an amount of approximately 5 to 35 % w/w in the core material, preferably 10 to 35 % w/w, or most preferably 15 to 35 % by weight calculated on the weight of the core material excluding the weight of the optional starter seed.

The alkalizing agent is selected from compounds like disodium hydrogen phosphate, trisodium phosphate, arginine or talc etc, provided that they give a pH of not less than 8.5 when measured in a 2% w/w water solution/dispersion with a pH-measuring electrode. At least one alkalizing agent has to be incorporated in the core material, but also any combinations of alkalizing agents can be used.

The swelling agent is selected among pharmaceutically acceptable disintegrants, preferably among crosslinked polyvinyl pyrrolidone, crosslinked sodium carboxymethylcellulose, sodium starch glycolate or low-substituted hydroxypropyl cellulose (L-HPC), alone or in any combinations. The amount of swelling agent is pre-determined to effectuate the start of dissolution of the core material at a proper time. Preferably, the core material comprises approximately 20 to 60 % by weight of the swelling agent calculated on the weight of the core material excluding any optional starting seed. More preferably a concentration of 25 to 55 % by weight, or especially 30 to 50 % by weight of the swelling agent calculated in the same manner.

Alternatively, the swelling agent or a portion of the swelling agent may optionally be prepared and incorporated in a separate layer. Such a separate layer will cover the core material and also comprise binders and optionally an alkalizing agent and/or pharmaceutically acceptable excipients.

Optionally, an osmotic agent is incorporated in the core material. Such an osmotic agent is watersoluble and will provide an osmotic pressure in the tablet. Examples of osmotic agents arc magnesium sulphate, sodium chloride, lithium chloride, potassium chloride. potassium sulphate, sodium carbonate, lithium sulphate, calcium bicarbonate, sodium sulphate, calcium lactate, urea, magnesium succinate, sucrose or mixtures thereof.

Alternatively, the active ingredient, optionally mixed with any of the components defined above, can be formulated into a core material. Said core material may be produced by extrusion/spheronization, balling or compression utilizing, different process equipments.

For extrusion/spheronization processes incorporation of a microcrystalline cellulose and a low-substituted hydroxypropylcellulose in the core material is preferred.

### Semipermeable membrane.

The membrane comprises a water insoluble polymer and a modifying additive and optionally pharmaceutically acceptable excipients like fillers, colorants etc. The excipients should be insoluble or hardly soluble in acidic solutions, or present in such amounts that they do not influence the solubility properties of the membrane.

Preferably, water insoluble polymer may be selected among semipermeable water insoluble polymers like ethylcellulose, cellulose acetate, polyvinyl acetate, and ammonio methacrylate copolymer type A and type B (Eudragit RL, Eudragit RS) etc.

The modifying agent in the semipermeable membrane may be a talc or fumed silica (e.g. Aerosil or Cab-O-Sil.). Preferably an alkaline reacting modifying agent such as talc is used.

The semipermeable membrane comprises an amount of modifying agent to water insoluble polymer on a weight to weight ratio of from 90:10 up to 50:50. Preferably the amount of modifying agent to water insoluble polymer on a weight to weight ratio is from 80:20 up to 60:40 in the membrane.

The core material will be layered with a sufficient amount of the semipermeable membrane composition to cover the core material. Preferably, the amount of semipermeable membrane applied is approximately 3-30% by weight of the weight of the core material. The amount of semipermeable membrane for a desired dosage form is adjusted to obtain a desired lagtime and an adequate dissolution.

### Final dosage form

The prepared core material coated with the semipermeable membrane is filled into a capsule (gelatine or HPMC capsule), or optionally mixed with tablet excipients and compressed into a multiple unit tableted dosage form. In the expression "tablet excipients" is also effervescent tablet excipients included when referring to multiple unit tablets.
Prepared tablets are optionally covered with filmforming agent(s) to obtain a smooth surface of the tablet and/or to further enhance the stability of the tablet during packaging and transport. Such a tablet coating layer may further comprise additives like anti-tacking agents, colorants and pigments or other additives to obtain a tablet of good appearance.

The claimed dosage forms are suitable for oral administration. The dose will depend on the nature and severity of the disease to be treated. The dose may also vary according to the age, body weight, and response of the individual patient. Children and patients with liver diseases as well as patients under long term treatment will generally benefit from doses that are somewhat lower than the average. In the treatment of severe conditions higher doses than average may be used.

Preferably, a dosage form comprising for instance 1 - 100 mg of omeprazole or S-omeprazole will be administered once a day. Suitable doses comprise preferably 10 - 80 mg. The dosage form may be administered together with other suitable drugs, such as antibacterial compound(s), NSAID(s), motility stimulating agents, and/or antacids.

### Examples

The following examples describe the invention more in detail.

### Example 1

Core materials in the form of pellets made by extrusion and spheronization.

The following compositions were used to prepare core materials;

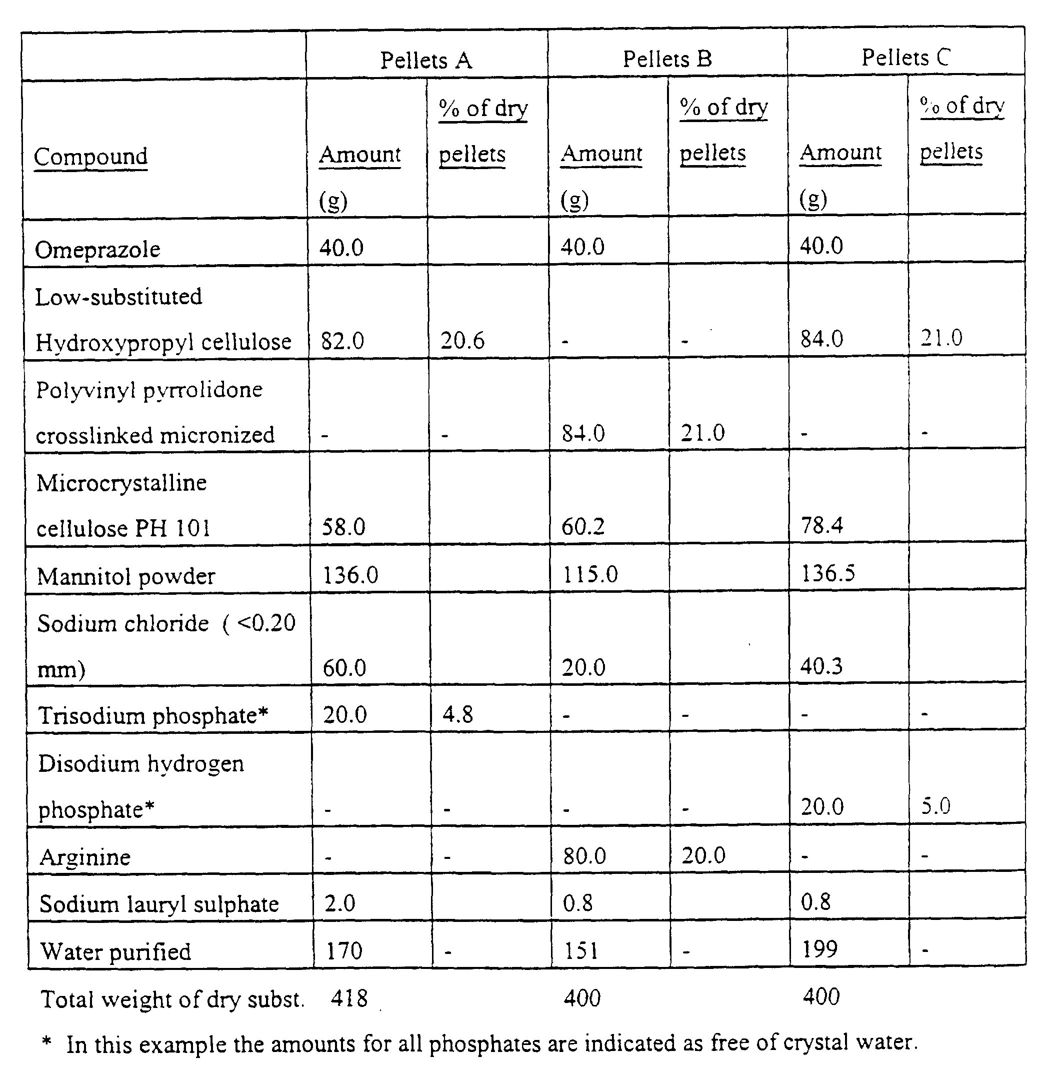

The powders were mixed and then wetted with the granulating solution. When needed extra water was added afterwards, until total amount added water corresponded to the value given in the table above. The wet mass was subjected for extrusion through a screen having 1.0 mm in diameter apertures. The strings obtained were shaped to pellets in a spheronizer operated at 350 rpm. The pellets were dried in a fluid bed apparatus with inlet air temperature set to 50 degrees Celsius.

Granulating liquid used for composition A was 2.72 g of the trisodium phosphate and all the sodium lauryl sulphate dissolved in 50 grams of the water.

Granulating liquid used for composition B was 10.0 g of the arginine and all the sodium lauryl sulphate dissolved in 100 grams of the water.

Granulating liquid used for composition C was 8.06 g of the disodium hydrogen phosphate and all the sodium lauryl sulphate dissolved in 100 grams of the water.

Remark: Only parts of composition B were possible to get through the extruder, however material for further experimentation was obtained.

### Example 2

Core material in the form of pellets prepared by layering technique.

A drug containing suspension was made according to the composition below;

| Compound | Amount |
|---|---|
| Omeprazole | 219 g |
| HPMC, 6 cps | 39.8 g |
| Disodiumhydrogen phosphate | 42.9 g |
| Polysorbate 80 | 4.8 g |
| Purified water | 919 g |

First the polysorbate 80 was dissolved in the water. Then the phosphate was dissolved during stirring. Then the HPMC was dissolved whereafter the drug was suspended in the obtained solution. The suspension was sprayed onto 150 g of sugar spheres (Non-pareil) in a fluidized bed. The weight of the obtained product was 355 g and the omeprazole content was 456 mg/g.

A suspension containing swellable substance was prepared according to the following composition;

| | | % |
|---|---|---|
| Cross-linked polyvinyl pyrrolidone micronized (Kollidon CL-M) | 187.8 g | 41* |
| Hydroxypropylcellulose L (HPC-L from Nisso) | 46.9 g | |
| Talc | 140.8 g | |
| EtOH (99.5%) | 1500 g | |

| | | |
|---|---|---|
| * % w/w of core material not including starter seed. | | |

HPC-L was dissolved in ethanol during stirring, then the talc and swelling agent Kollidon CL-M was added. The suspension was sprayed onto 130 g of the drug-layered spheres as prepared above in a Wurster equipped fluidized bed until the omeprazole content of the obtained core material was 130 mg/g. The weight of the obtained product was 455 g.

### Example 3

### Membrane coated pellets.

The core material from Example 2 was coated in a fluid bed apparatus with an ethyl cellulose solution having talc suspended therein. The composition of the suspension used was:

| Substance | Amount | % of dry membrane |
|---|---|---|
| Ethyl cellulose N-10 | 13.5 parts | 30% |
| Ethanol (99.5% ) | 1455 parts | - |
| Talc | 31.5 parts | 70% |
| Total | 1500 parts | 100% |

80 grams of core material from example 2 was coated with this suspension until the omeprazole content was 107 mg/g.

### Example 4

### Test of the prepared membrane coated pellets.

The prepared membrane coated core material was tested for gastric acid resistance and dissolution as described below.

### Test for gastric acid resistance

The pellets were tested for gastric acid resistance by immersing them in 0.1 M HCl for 2 hrs and the determining the remaining drug fraction. The fluid phase (the HCl) had an addition of 0.1 g/liter of sodium lauryl sulphate as wetting agent. The remaining drug fraction was 96%.

### Test for dissolution

Dissolution of active substance was tested accordingly, first pellets were immersed in the test-fluid described above for 2 hrs, then buffer components (phosphate salts) were added to change the pH to 6.8.
Samples of the dissolution medium were withdrawn and analyzed with HPLC at the given time intervals. Results;

| Time, Hrs (after 2hrs of pre-exposure in acid medium) | % Dissolved |
|---|---|
| 0.5 | 3 |
| 1 | 18 |
| 2 | 60 |
| 3 | 73 |

## Claims

1. An oral dosage form, comprising a core material coated with a semipermeable membrane, comprising a water-insoluble polymer and talc or fumed silica, said semipermeable membrane being able to disrupt, and wherein the core material comprises an active ingredient selected from the group of omeprazole, an alkaline salt thereof, *S*-omeprazole and an alkaline salt thereof, in admixture with one or more alkalizing agents, one or more swelling agents, and optionally pharmaceutically acceptable excipients; wherein the talc or fumed silica and water insoluble polymer are in a weight ratio of between 90:10 and 50:50, and which dosage form is not enteric coated.

2. A dosage form according to claim 1, wherein the talc or fumed silica and water insoluble polymer are in a weight ratio of between 80:20 and 60:40.

3. A dosage form according to claim 1, wherein the active ingredient is omeprazole.

4. A dosage form according to claim 1, wherein the active ingredient is a magnesium salt of omeprazole having a crystallinity of more than 70% as determined by X-ray powder diffraction.

5. A dosage form according to claim 1, wherein the active ingredient is a magnesium salt of *S*-omeprazole.

6. A dosage form according to any one of claims 1-5, wherein the core material comprises a sugar sphere layered with a suspension or solution of the active ingredient, one or more alkaline additives, one or more swelling agents and optionally pharmaceutically acceptable excipients.

7. A dosage form according to any one of claims 1-6, wherein the dosage form comprises individual pellets of the core material coated with the semipermeable membrane.

8. A dosage form according to any one of claims 1-7, wherein the core material further comprises an osmotic agent.

9. A dosage form according to any one of claims 1-8, wherein the alkalizing agent is selected from the group of compounds that give a pH of not less than 8.5 when measured in a 2% w/w water solution/dispersion with a pH-measuring electrode.

10. A dosage form according to any one of claims 1-9, wherein the alkalizing agent is an agent selected from the group consisting of disodium hydrogen phosphate, trisodium phosphate, arginine and talc.

11. A dosage form according to any one of claims 1-10, wherein the alkalizing agent is present in an amount of approximately 5 to 35% by weight of the core material excluding the weight of an optional sugar sphere.

12. A dosage form according to claim 11, wherein the alkalizing agent is present in an amount of 15 to 35% by weight of the core material excluding the weight of an optional sugar sphere.

13. A dosage form according to any one of claims 1-12, wherein the swelling agent is selected from the group of crosslinked polyvinyl pyrrolidone, crosslinked sodium carboxymethylcellulose, sodium starch glycolate and low-substituted hydroxypropyl cellulose (L-HPC).

14. A dosage form according to any one of claims 1-13, wherein the swelling agent is present in an amount of 20 to 60% by weight of the core material excluding the weight of an optional sugar sphere.

15. A dosage form according to claim 14, wherein the swelling agent is present in an amount of 30 to 50% by weight of the core material excluding the weight of an optional sugar sphere.

16. A dosage form according to any one of claims 1-15, wherein the water insoluble polymer is selected from the group of ethylcellulose, cellulose acetate, polyvinyl acetate, and ammonio methacrylate copolymer type A and type B.

17. A dosage form according to any one of claims 1-16, wherein the water insoluble polymer is present in an amount of approximately 3-30% by weight of the core material.

18. A process for the manufacture of a dosage form as defined in claim 1, wherein a core material comprising an active ingredient selected from the group of omeprazole, an alkaline salt thereof, *S*-omeprazole and an alkaline salt thereof, in admixture with one or more alkaline additives, one or more swelling agents, and optionally pharmaceutically acceptable excipients is formed, the core material is coated with a semipermeable membrane being able to disrupt and which dosage form has no enteric coating.

19. Use of an oral pharmaceutical dosage form according to any of claims 1-17 in the manufacture of a medicament with improved inhibition of gastric acid secretion.

20. Use of an oral pharmaceutical dosage form according to any of claims 1-17 in the manufacture of a medicament with improved therapeutic effect in the treatment of gastrointestinal disorders associated with excess acid secretion.

21. An oral dosage form according to any one of claims 1-17 filled into a capsule.

22. An oral dosage form according to any one of claims 1-17 optionally mixed with tablet excipients, said dosage form being compressed into a multiple unit tableted dosage form.

## Patentansprüche

1. Orale Dosisform, enthaltend ein mit einer ein wasserunlösliches Polymer und Talk oder pyrogene Kieselsäure enthaltenden semipermeablen Membran, die zerreißen kann, beschichtetes Kernmaterial, welches einen Wirkstoff aus der Gruppe bestehend aus Omeprazol, einem alkalischen Salz davon, *S*-Omeprazol und einem alkalischen Salz davon in Abmischung mit einem oder mehreren Alkalisierungsmitteln, einem oder mehreren Quellmitteln und gegebenenfalls pharmazeutisch annehmbaren Trägerstoffen enthält; wobei der Talk bzw. die pyrogene Kieselsäure und das wasserunlösliche Polymer in einem Gewichtsverhältnis zwischen 90:10 und 50:50 vorliegen und die Dosisform nicht magensaftresistent beschichtet ist.

2. Dosisform nach Anspruch 1, bei der der Talk bzw. die pyrogene Kieselsäure und das wasserunlösliche Polymer in einem Gewichtsverhältnis zwischen 80:20 und 60:40 vorliegen.

3. Dosisform nach Anspruch 1, bei der es sich bei dem Wirkstoff um Omeprazol handelt.

4. Dosisform nach Anspruch 1, bei der es sich bei dem Wirkstoff um ein Magnesiumsalz von Omeprazol mit einer mittels Röntgenpulverbeugung bestimmten Kristallinität von mehr als 70% handelt.

5. Dosisform nach Anspruch 1, bei der es sich bei dem Wirkstoff um ein Magnesiumsalz von *S*-Omeprazol handelt.

6. Dosisform nach einem der Ansprüche 1-5, bei der das Kernmaterial ein mit einer Suspension oder Lösung des Wirkstoffs beschichtetes Zuckerkügelchen, ein oder mehrere alkalische Additive, ein oder mehrere Quellmittel und gegebenenfalls pharmazeutisch annehmbare Trägerstoffe enthält.

7. Dosisform nach einem der Ansprüche 1-6, die einzelne Pellets aus dem Kernmaterial, die mit der semipermeablen Membran beschichtet sind, enthält.

8. Dosisform nach einem der Ansprüche 1-7, bei der das Kernmaterial ferner ein osmotisches Mittel enthält.

9. Dosisform nach einem der Ansprüche 1-8, bei der das Alkalisierungsmittel aus der Gruppe bestehend aus verbindungen, die bei Messung in einer 2 gew.-%igen wäßrigen Lösung/Dispersion mit einer pH-Meßelektrode einen pH-Wert von mindestens 8,5 ergeben, stammt.

10. Dosisform nach einem der Ansprüche 1-9, bei der es sich bei dem Alkalisierungsmittel um ein Mittel aus der Gruppe bestehend aus Dinatriumhydrogenphosphat, Trinatriumphosphat, Arginin und Talk handelt.

11. Dosisform nach einem der Ansprüche 1-10, bei der das Alkalisierungsmittel in einer Menge von ungefähr 5 bis 35 Gew.-%, bezogen auf das Kernmaterial unter Ausschluß des Gewichts eines fakultativen Zuckerkügelchens, vorliegt.

12. Dosisform nach Anspruch 11, bei der das Alkalisierungsmittel in einer Menge von 15 bis 35 Gew.-%, bezogen auf das Kernmaterial unter Ausschluß des Gewichts eines fakultativen Zuckerkügelchens, vorliegt.

13. Dosisform nach einem der Ansprüche 1-12, bei der das Quellmittel aus der Gruppe bestehend aus vernetztem Polyvinylpyrrolidon, vernetzter Natriumcarboxymethylcellulose, Natriumstärkeglykolat und niedrig substituierter Hydroxypropylcellulose (L-HPC) stammt.

14. Dosisform nach einem der Ansprüche 1-13, bei der das Quellmittel in einer Menge von 20 bis 60 Gew.-%, bezogen auf das Kernmaterial unter Ausschluß des Gewichts eines fakultativen Zuckerkügelchens, vorliegt.

15. Dosisform nach Anspruch 14, bei der das Quellmittel in einer Menge von 30 bis 50 Gew.-%, bezogen auf das Kernmaterial unter Ausschluß des Gewichts eines fakultativen Zuckerkügelchens, vorliegt.

16. Dosisform nach einem der Ansprüche 1-15, bei der das wasserunlösliche Polymer aus der Gruppe bestehend aus Ethylcellulose, Celluloseacetat, Polyvinylacetat und Ammoniummethacrylat-Copolymer Typ A und B stammt.

17. Dosisform nach einem der Ansprüche 1-16, bei der das wasserunlösliche Polymer in einer Menge von ungefähr 3-30 Gew.-%, bezogen auf das Kernmaterial, vorliegt.

18. Verfahren zur Herstellung einer Dosisform gemäß Anspruch 1, bei dem man ein Kernmaterial, das einen Wirkstoff aus der Gruppe bestehend aus Omeprazol, einem alkalischen Salz davon, *S*-Omeprazol und einem alkalischen Salz davon in Abmischung mit einem oder mehreren Alkalisierungsmitteln, einem oder mehreren Quellmitteln und gegebenenfalls pharmazeutisch annehmbaren Trägerstoffen bildet und das Kernmaterial mit einer semipermeablen Membran, die zerreißen kann, beschichtet, wobei die Dosisform keine magensaftresistente Beschichtung aufweist.

19. Verwendung einer oralen pharmazeutischen Dosisform gemäß einem der Ansprüche 1-17 bei der Herstellung eines Arzneimittels mit verbesserter Hemmung der Magensäuresekretion.

20. Verwendung einer oralen pharmazeutischen Dosisform gemäß einem der Ansprüche 1-17 bei der Herstellung eines Arzneimittels mit verbesserter therapeutischer Wirkung bei der Behandlung von mit übermäßiger Magensäuresektretion einhergehenden gastrointestinalen Störungen.

21. Orale Dosisform nach einem der Ansprüche 1-17, die in eine Kapsel gefüllt ist.

22. Orale Dosisform nach einem der Ansprüche 1-17, gegebenenfalls in Abmischung mit Tablettenträgerstoffen, die zu einer tablettierten Multiple-Unit-Dosisform verpreßt wird.

## Revendications

1. Forme de dosage orale, comprenant un matériau de coeur enrobé d'une membrane semi-perméable, comprenant un polymère insoluble dans l'eau et du talc ou de la silice pyrogénée, ladite membrane semi-perméable étant capable de se rompre, et dans laquelle le matériau de coeur comprend un ingrédient actif choisi parmi le groupe constitué par l'oméprazole, un sel alcalin de celui-ci, le *S*-oméprazole et un sel alcalin de celui-ci, en co-mélange avec un ou plusieurs agents alcalinisants, un ou plusieurs agents gonflants, et éventuellement des excipients pharmaceutiquement acceptables ; dans laquelle le talc ou la silice pyrogénée et le polymère insoluble dans l'eau sont présents en un rapport pondéral compris entre 90:10 et 50:50, et laquelle forme de dosage n'est pas à enrobage gastro-résistant.

2. Forme de dosage selon la revendication 1, dans laquelle le talc ou la silice pyrogénée et le polymère insoluble dans l'eau sont présents en un rapport pondéral compris entre 80:20 et 60:40.

3. Forme de dosage selon la revendication 1, dans laquelle l'ingrédient actif est l'oméprazole.

4. Forme de dosage selon la revendication 1, dans laquelle l'ingrédient actif est un sel de magnésium de l'oméprazole, ayant une cristallinité supérieure à 70% telle que déterminée par diffraction de poudre aux rayons X.

5. Forme de dosage selon la revendication 1, dans laquelle l'ingrédient actif est un sel de magnésium du *S*-oméprazole.

6. Forme de dosage selon l'une quelconque des revendications 1-5, dans laquelle le matériau de coeur comprend une sphère en sucre recouverte d'une couche de suspension ou de solution de l'ingrédient actif, d'un ou plusieurs additifs alcalins, d'un ou plusieurs agents gonflants et éventuellement d'excipients pharmaceutiquement acceptables.

7. Forme de dosage selon l'une quelconque des revendications 1-6, dans laquelle la forme de dosage comprend des pastilles individuelles du matériau de coeur enrobé de la membrane semi-perméable.

8. Forme de dosage selon l'une quelconque des revendications 1-7, dans laquelle le matériau de coeur comprend en outre un agent osmotique.

9. Forme de dosage selon l'une quelconque des revendications 1-8, dans laquelle l'agent alcalinisant est choisi parmi le groupe de composés donnant un pH non inférieur à 8,5 lors de sa mesure dans une solution/dispersion d'eau à 2% p/p à l'aide d'une électrode de mesure du pH.

10. Forme de dosage selon l'une quelconque des revendications 1-9, dans laquelle l'agent alcalinisant est un agent choisi parmi le groupe constitué par l'hydrogénophosphate disodique, le phosphate trisodique, l'arginine et le talc.

11. Forme de dosage selon l'une quelconque des revendications 1-10, dans laquelle l'agent alcalinisant est présent en une quantité d'environ 5 à 35% en poids du matériau de coeur excluant le poids d'une sphère en sucre éventuelle.

12. Forme de dosage selon la revendication 11, dans laquelle l'agent alcalinisant est présent en une quantité de 15 à 35% en poids du matériau de coeur excluant le poids d'une sphère en sucre éventuelle.

13. Forme de dosage selon l'une quelconque des revendications 1-12, dans laquelle l'agent gonflant est choisi parmi le groupe constitué par la polyvinylpyrrolidone réticulée, la carboxyméthyl cellulose de sodium réticulée, le glycolate d'amidon sodique et l'hydroxypropyl cellulose à faible degré de substitution (L-HPC).

14. Forme de dosage selon l'une quelconque des revendications 1-13, dans laquelle l'agent gonflant est présent en une quantité de 20 à 60% en poids du matériau de coeur excluant le poids d'une sphère en sucre éventuelle.

15. Forme de dosage selon la revendication 14, dans laquelle l'agent gonflant est présent en une quantité de 30 à 50% en poids du matériau de coeur excluant le poids d'une sphère en sucre éventuelle.

16. Forme de dosage selon l'une quelconque des revendications 1-15, dans laquelle le polymère insoluble dans l'eau est choisi parmi le groupe constitué par l'éthylcellulose, l'acétate de cellulose, l'acétate de polyvinyle, et un copolymère d'ammoniométhacrylate de type A et de type B.

17. Forme de dosage selon l'une quelconque des revendications 1-16, dans laquelle le polymère insoluble dans l'eau est présent en une quantité d'environ 3-30% en poids du matériau de coeur.

18. Procédé de fabrication d'une forme de dosage telle que définie selon la revendication 1, dans lequel un matériau de coeur comprenant un ingrédient actif choisi parmi le groupe constitué par l'oméprazole, un sel alcalin de celui-ci, le *S*-oméprazole et un sel alcalin de celui-ci, en co-mélange avec un ou plusieurs additifs alcalins, un ou plusieurs agents gonflants, et éventuellement des excipients pharmaceutiquement acceptables, est formé, le matériau de coeur est enrobé d'une membrane semi-perméable capable de se rompre, et laquelle forme de dosage ne possède pas d'enrobage gastro-résistant.

19. Utilisation d'une forme de dosage pharmaceutique orale selon l'une quelconque des revendications 1-17 dans la fabrication d'un médicament ayant une inhibition améliorée de la sécrétion de l'acide gastrique.

20. Utilisation d'une forme de dosage pharmaceutique orale selon l'une quelconque des revendications 1-17 dans la fabrication d'un médicament ayant un effet thérapeutique amélioré dans le traitement de troubles gastro-intestinaux associés à une sécrétion excessive d'acide.

21. Forme de dosage orale selon l'une quelconque des revendications 1-17, chargée dans une capsule.

22. Forme de dosage orale selon l'une quelconque des revendications 1-17, éventuellement mélangée avec des excipients pour comprimés, ladite forme de dosage étant comprimée en une forme de dosage comprimée à unités multiples.
